(19) Europäisches Patentamt
European Patent Office
Office européen des brevets

(11) Veröffentlichungsnummer: **0 242 328 B1**

(12) # EUROPÄISCHE PATENTSCHRIFT

(45) Veröffentlichungstag der Patentschrift: **24.07.91**

(51) Int. Cl.5: **A61K 31/195**, A61K 47/00

(21) Anmeldenummer: **87810220.1**

(22) Anmeldetag: **08.04.87**

(54) **Arzneimittel zur Behandlung von Entzündungen im Auge.**

(30) Priorität: **14.04.86 DE 3612537**

(43) Veröffentlichungstag der Anmeldung:
**21.10.87 Patentblatt 87/43**

(45) Bekanntmachung des Hinweises auf die Patenterteilung:
**24.07.91 Patentblatt 91/30**

(84) Benannte Vertragsstaaten:
**BE CH ES FR GB GR IT LI LU NL SE**

(56) Entgegenhaltungen:
**DE-A- 3 026 402**
**GB-A- 2 059 768**

**CHEMICAL ABSTRACTS, Band 100, Nr. 2, January 1984, Seite 370, Zusammenfassung Nr. 12683w, Columbus, Ohio, US; & JP-A-58 174 310 (WAKAMOTO PHARMACEUTICAL CO., LTD) 13-10-1983**

(73) Patentinhaber: **DISPERSA AG**
**Riethofstrasse 1**
**CH-8442 Hettlingen(CH)**

(72) Erfinder: **Doulakas, Johann, Dr.**
**Landenbergstrasse 33**
**CH-8404 Winterthur(CH)**

(74) Vertreter: **Schluep, Hans-Peter et al**
**c/o CIBA GEIGY AG Patentabteilung Postfach**
**CH-4002 Basel(CH)**

**EP 0 242 328 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Arzneimittel zur lokalen Behandlung von Enzündungen im Auge, das Diclofenac-Natrium als Wirkstoff enthält und das eine erhöhte Stabilität der Formulierung und eine sehr gute Verträglichkeit im Auge aufweist.

Für die Behandlung stärkerer akuter oder chronisch rezidivierender Entzündungserscheinungen am Auge werden bisher hauptsächlich Kortikosteroide eingesetzt. Die immunsupressive Wirkung dieser Substanzen birgt allerdings die Gefahr einer Verschlimmerung des Krankheitsbildes durch eine bakterielle oder virale Infektion in sich. Die Entwicklung und Einführung von nichtsteroidalen Entzündungshemmern in die ophthalmologische Therapie ist deshalb in letzter Zeit forciert worden.

Diclofenac-Natrium mit der chemischen Bezeichnung Natrium-2-(2,6-dichloranilino)-phenylacetat ist ein bekannter nicht-steroidaler Entzündungshemmer; vgl. DE-C 1 543 639 und DE-C 1 793 592. Die Applikationsformen umfassen sämtliche Formen von Tabletten, Kapseln und Dragees sowie Suppositorien und Ampullen.

Der Wirkstoff wurde bisher hauptsächlich in der Otorhinolaryngologie, Gynäkologie, Urologie, Pädiatrie und Rheumatologie eingesetzt. Daneben hat die Substanz aber auch systemische Anwendung in der Ophthalmologie gefunden. Ein Nachteil dieser Anwendung liegt darin, dass über den systemischen Weg am Wirkort nur relativ geringe Wirkspiegel erzielt werden, wobei nicht davon ausgegangen werden kann, dass eine Dossierhöhung auch zu einer entsprechenden lokalen Wirkspiegelerhöhung führt.

Die topische Anwendung von Diclofenac-Natrium am Auge sollte hier Vorteile in zweierlei Hinsicht bringen: Erstens wird nicht der gesamte Organismus mit der Wirksubstanz belastet, um einen lokalen Effekt zu erzielen, und zweitens wird mit einer Augentropfenlösung ein lokal höherer Wirkspiegel erreicht.

Eine Diclofenac-Natrium enthaltende Lösung zur Anwendung am Auge ist aus der JP-A-58/174310 bekannt. Die dort beschriebenen Augentropfen enthalten Diclofenac-Natrium in einer Konzentration von 0,01 bis 1 %. Sie weisen einen pH-Wert von vorzugsweise 7 bis 8 auf. Als Puffer werden Phosphate, Borsäure, Borax und organische Säuren verwendet. Natriumchlorid und Mannit werden als Isotonisierungszusätze genannt. Als Lösungsvermittler werden Polyoxyethylensorbitanmonooleat, Polyoxyethylen-oxystearinsäuretriglycerid, Polyoxyethylen-glykol und $\alpha$- und $\beta$-Cyclodextrin erwähnt. Als Verdickungsmittel sind Polyvinylpyrrolidon, Methylcellulose, Hydroxypropylmethylcellulose und Hydroxypropylcellulose angegeben. Als Konservierungsmittel werden ferner Benzalkoniumchlorid, Cetylpyridiniumchlorid, Chlorobutanol und Thiomersal genannt. Schliesslich werden zur Verminderung der Augenreizung pharmakologisch verträgliche Calcium- oder Magnesiumsalze, gewöhnlich in einer Menge von 0,3 bis 2 Mol pro Mol Wirkstoff, zugesetzt.

Der Zusatz von Calcium- oder Magnesiumsalzen von physiologisch verträglichen Säuren zur Verminderung der Augenreizung durch entzündungshemmende Augentropfen, die als Wirkstoff einen nicht-steroidalen Entzündungshemmer mit einer Carboxylgruppe im Molekül enthalten, ist ferner in der JP-A-58/174309 beschrieben.

Die in den JP-A-58/174310 und 58/174309 beschriebenen Formulierungen für Augentropfen sind jedoch mit wesentlichen Nachteilen behaftet, die sie zur Verwendung als Fertigarzneimittel ungeeignet machen.

Ein wesentlicher Nachteil der bekannten Formulierungen besteht darin, dass sie in den beschriebenen Zusammensetzungen unstabil sind.

Bei Augentropfen ist ferner in den meisten europäischen und anderen Ländern eine Konservierung der Präparate vorgeschrieben, d.h. ein Schutz gegen einen Befall und eine anschliessende Vermehrung von Mikroorganismen. In der JP-A-58/174310 werden zwar Angaben zur möglichen Verwendung von Konser vierungsmitteln gemacht, die angegebenen Konservierungsmittel sind jedoch für den genannten Zweck in den beschriebenen Formulierungen aus folgenden Gründen ungeeignet: Benzalkoniumchlorid und Cetylpyridiniumchlorid sind mit Diclofenac-Natrium ohne Zusatz eines passenden Lösungsvermittlers nicht kompatibel; ihr Zusatz führt zu einer Ausfällung in dem Präparat. Darüber hinaus ist Benzalkoniumchlorid mit Polyoxysorbaten, wie sie in der JP-A-58/174310 als Lösungsvermittler vorgeschlagen werden, inkompatibel. Chlorobutanol ist nur bei einem pH-Wert unter 6 stabil und eignet sich deshalb nicht zur Verwendung in Präparaten, die einen pH-Wert von 7 bis 8 aufweisen. Das in der JP-A-58/174310 ausserdem noch als Konservierungsmittel genannte Thiomersal ist nicht kompatibel mit dem in den beschriebenen Formulierungen zur Herstellung von Isotonie enthaltenen Kochsalz. Ausserdem ist das Thiomersal selbst in wässriger Lösung unstabil. Die Formulierungsbeispiele der JP-A-58/174310 sind ohne ein Konservierungsmittel abgefasst.

Ein weiterer Nachteil der bekannten Formulierungen besteht darin, dass Calcium- oder Magnesiumsalze zur Verhinderung von Augenreizungen durch den Wirkstoff oder durch das als Lösungsvermittler bevorzugt eingesetzte $\beta$-Cyclodextrin zugesetzt werden müssen. Der Zusatz von Calcium- und Magnesiumsalzen zu den Formulierungen erweist sich als unerwünscht, da Komplexbildung der Erdalkaliionen mit dem Wirkstoff

auftreten kann, wodurch dessen Verfügbarkeit und Stabilität beeinträchtigt wird.

Aus den genannten Gründen können somit nach dem Stand der Technik keine Diclofenac-Natrium enthaltenden Augentropfen, die stabil und den gesetzlichen Anforderungen entsprechend konserviert sind sowie das Auge nicht reizen, erhalten werden.

Der Erfindung liegt deshalb die Aufgabe zugrunde, Diclofenac-Natrium als Augentropfen zu formulieren, die eine für ein Fertigarzneimittel ausreichende Stabilität aufweisen und die den Anforderungen entsprechend konserviert und im Auge gut verträglich sind.

Diese Aufgabe wird durch den überraschenden Befund gelöst, dass durch den Einsatz von bestimmten Stabilisatoren einerseits eine ausreichende Stabilisierung des Wirkstoffs erreicht werden kann und andererseits gleichzeitig die Verwendung eines Konservierungsmittels möglich wird, da die mit dessen Verwendung verbundenen Stabilitäts-und Kompatibilitätsprobleme gelöst werden. Schliesslich hat sich auch gezeigt, dass in der hier beschriebenen Erfindung die Notwendigkeit, Calcium- oder Magnesiumsalze zur Beseitigung von Augenreizungen einzusetzen, entfällt.

Gegenstand der Erfindung ist somit ein ausreichend stabiles, konserviertes, gut verträgliches und wirksames Arzneimittel zur Behandlung von Entzündungen im Auge, enthaltend eine wässrige Lösung von Diclofenac-Natrium, einen Puffer, ein Isotonisierungsmittel, einen Lösungsvermittler und ein Konservierungsmittel. Das Arzneimittel ist dadurch gekennzeichnet, dass es als Stabilisator für den Wirkstoff und das Konservierungsmittel ein Aminopolyol enthält.

Durch den erfindungsgemässen Zusatz eines speziellen Stabilisators zu der wässrigen Diclofenac-Natrium-Zubereitung, die zur Verwendung als Augentropfen bestimmt ist, werden die mit den bekannten, Diclofenac-Natrium enthaltenden Augentropfen verbundenen Schwierigkeiten beseitigt.

Als besonders geeignet für den angestrebten Zweck haben sich 2-Amino-2-hydroxymethyl-1,3-propandiol (Trometamol) und seine Homologen mit bis zu 10 C-Atomen, insbesondere 5 bis 7 C-Atomen, erwiesen.

Trometamol und seine Homologen mit bis zu 10, bevorzugt 5 bis 7, C-Atomen lassen sich auch durch die allgemeine Formel I veranschaulichen:

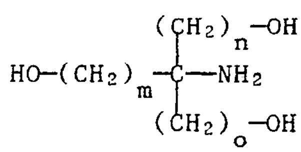

(I)

m, n, o (unabhängig voneinander ) ≙ eine ganze Zahl, mindestens 1
Summe aus m, n und o ≙ 3 bis 9, bevorzugt 3 bis 6
In erster Linie bevorzugt ist 2-Amino-2-hydroxymethyl-1,3-propandiol (Trometamol), das einer Verbindung der Formel I mit m = n = o = 1 entspricht.

Durch den Zusatz dieser Stabilisatoren wird die sonst im Laufe der Lagerung auftretende Zersetzung des Diclofenac-Natrium wirksam verhindert. Ausserdem wird die Verwendung eines Konservierungsmittels ermöglicht, das zur Verhinderung eines Befalls des Präparates durch Mikroorganismen an sich erforderlich und gesetzlich vorgeschrieben ist. Insbesondere kann das in wässriger Lösung an sich unstabile Thiomersal, das besonders günstige Konservierungseigenschaften aufweist, in der Formulierung der Erfindung eingesetzt werden, da es durch den verwendeten Stabilisator indirekt mitstabilisiert wird. Der Stabilisator verhindert eine Adsorption des Thiomersals an der Behälterwand, wodurch dessen Haltbarkeit entscheidend verbessert wird. Schliesslich hat sich gezeigt, dass in der Formulierung der Erfindung keine Augenreizung auftritt, so dass der Zusatz der wegen des Wirkstoffs unerwünschten Calcium- oder Magnesiumsalze nicht erforderlich ist.

Die einzelnen Bestandteile des Augenarzneimittels der Erfindung werden nachstehend erläutert.

Der Wirkstoff Diclofenac-Natrium wird in einer Konzentration von 0,01 bis 0,15 %, vorzugsweise 0,05 bis 0,11 %, eingesetzt. Besonders bevorzugt ist eine Wirkstoffkonzentration von etwa 0,1 %.

Der pH-Wert der Formulierung beträgt vorzugsweise 6,8 bis 7,5. Als Puffersubstanzen können beispielsweise Borsäure, Borate, Phosphate und organische Säuren eingesetzt werden. Die Verwendung von Borsäure ist bevorzugt, wobei der zugesetzte Stabilisator, beispielsweise das Trometamol, als basische Komponente des Puffergemisches wirkt.

Die als Puffersubstanz eingesetzte Borsäure wird vorzugsweise in derartiger Menge verwendet, dass damit gleichzeitig Isotonie der Formulierung erreicht wird. Gegebenenfalls können als Isotonisierungsmittel auch Glucose, Citrate, Phosphate, Borate oder andere bekannte Stoffe eingesetzt werden.

Natriumchlorid wird bei Verwendung von Thiomersal als Konservierungsmittel nicht zur Isotonisierung des Präparates verwendet, um Kompatibilitätsschwierigkeiten mit dem Thiomersal zu vermeiden. Andererseits kann Natriumchlorid jedoch eingesetzt werden, wenn Benzalkoniumchlorid oder Cetylpyridiniumchlorid als Konservierungsmittel verwendet werden.

Als Lösungsvermittler für das Diclofenac-Natrium werden in den Präparaten der Erfindung z.B. Fettsäureglycerin-Polyglykolester, Fettsäurepolyglykolester, Polyethylenglykole, Glycerinether oder Gemische aus diesen Verbindungen verwendet. Ein spezielles Beispiel für einen besonders bevorzugten Lösungsvermittler sind Reaktionsprodukte aus Rizinusöl und Ethylenoxid, beispielsweise das Handelsprodukt Cremophor EL®. Reaktionsprodukte aus Rizinusöl und Ethylenoxid haben sich als besonders gute Lösungsvermittler mit einer ausgezeichneten Augenverträglichkeit erwiesen. Die eingesetzte Konzentration richtet sich in erster Linie nach der Konzentration des Wirkstoffs. Es ist mindestens soviel zuzusetzen, dass der Wirkstoff in Lösung gebracht wird. Zum Beispiel beträgt die Konzentration des Lösungsvermittlers das 1- bis 100fache, insbesondere das 5- bis 60fache, der Wirkstoffkonzentration.

Ein Konservierungsmittel muss in den Präparaten der Erfindung enthalten sein, um einen Befall durch Mikroorganismen während des Anwendungszeitraums zu verhindern. Wegen seiner hervorragenden Konservierungseigenschaften ist dafür das Natriumsalz von 2-(Ethylmercurithio)-benzoesäure (Thiomersal) besonders bevorzugt. Es wird in einer Konzentration von 0,002 bis 0,02 %, vorzugsweise 0,002 bis 0,005 % und insbesondere von etwa 0,004 %, eingesetzt. Daneben können auch andere bekannte Konservierungsmittel, wie Benzalkoniumchlorid oder Cetylpyridiniumchlorid, in einer Konzentration von 0,005 bis 0,02 % verwendet werden. Es eignen sich auch Kombinationen der genannten Stoffe mit dem Dinatriumsalz von Edetinsäure.

Der Stabilisator wird dem Präparat der Erfindung in einer Menge von 0,05 bis 5 %, vorzugsweise 0,1 bis 1,0 %, zugesetzt. In dieser Menge bewirkt der Stabilisator, wie erwähnt, sowohl eine Stabilisierung des Wirkstoffs gegen chemischen Zerfall als auch eine Stabilisierung des Konservierungsmittels Thiomersal gegen dessen Zersetzung in wässriger Lösung. Auch die Kompatibilität der anderen erfindungsgemäss verwendbaren Konservierungsmittel Benzalkoniumchlorid und Cetylpyridiniumchlorid mit dem Wirkstoff wird durch den Stabilisator zusätzlich gewährleistet. Gegebenenfalls muss bei Verwendung dieser Konservierungsmittel die Menge des Lösungsvermittlers etwas erhöht werden.

Das Präparat der Erfindung wird mit Wasser für Injektionszwecke bereitet. Es wird eine Osmolalität von etwa 0,9 (301 mOsmol/kg) eingestellt.

Das Präparat der Erfindung lässt sich infolge seiner hervorragenden chemischen Stabilität über längere Zeit auch bei Raumtemperatur lagern und erfüllt die Anforderungen, die an ein derartiges Präparat während des therapeutischen Gebrauchs hinsichtlich seiner Haltbarkeit gestellt werden.

Das Arzneimittel der Erfindung wird in an sich bekannter Weise dadurch hergestellt, dass alle Komponenten des Arzneimittels unter sterilen Bedingungen homogen vermischt und abgefüllt werden.

Die folgenden Formulierungsbeispiele erläutern die Erfindung:

Beispiel 1

| Bestandteil | Menge |
|---|---|
| Diclofenac-Natrium | 0,1 % |
| 2-(Ethylmercurithio)-benzoesäure, Natriumsalz | 0,004 % |
| Borsäure | 1,9 % |
| Trometamol | 0,6 % |
| Cremophor EL® | 5,0 % |
| Wasser für Injektionszwecke | Restmenge |

Beispiel 2

| Bestandteil | Menge |
|---|---|
| Diclofenac-Natrium | 0,1 % |
| Benzalkoniumchlorid | 0,01 % |
| Borsäure | 1,9 % |
| Trometamol | 0,6 % |
| Cremophor EL® | 5,0 % |
| Wasser für Injektionszwecke | Restmenge |

Beispiel 3

| Bestandteil | Menge |
|---|---|
| Diclofenac-Natrium | 0,1 % |
| Cetylpyridiniumchlorid | 0,01 % |
| Borsäure | 1,9 % |
| Trometamol | 0,6 % |
| Cremophor EL® | 5,0 % |
| Wasser für Injektionszwecke | Restmenge |

Die Formulierungen der Beispiele 1, 2 und 3 sind bei Raumtemperatur 3-5 Jahre haltbar.

Die Verträglichkeit der Augentropfen wurde in einer Augenreizwirkungs- und Toxizitätsstudie untersucht. Die untersuchte Augentropfen-Lösung entspricht dem Formulierungsbeispiel 1. Die Testlösung wird über vier Wochen fünfmal pro Tag in den Bindehautsack appliziert. Für die Studie wird mit zwei Gruppen von je sechs Kaninchen gearbeitet.

Das folgende Versuchsschema kommt zur Anwendung:

| Gruppe | männlich | weiblich | rechtes Auge | linkes Auge |
|---|---|---|---|---|
| 1 | 3 (389-391)*) | 3 (395-397)*) | unbehandelt | Physiologische Kochsalzlösung |
| 2 | 3 (392-394)*) | 3 (398-400)*) | Augentropfen ohne Wirkstoff | Augentropfen mit 0,1 % Diclofenac-Na |

*) Nummer des Tieres

Im Hinblick auf Augenreizungen werden die Tiere zweimal pro Tag, vor der ersten und nach der letzten täglichen Applikation untersucht.

Als Ergebnis der Untersuchung kann Folgendes zusammengefasst werden:

Bei sämtlichen Kaninchen zeigen sich nach der vierwöchigen Behandlung mit 50 µl der 0,1%igen Diclofenac-Na-Augentropfenlösung weder lokal reizende noch systemische Symptome.

Als spezielle Untersuchungsmethode zur Erfassung von vermehrtem Tränenfluss infolge lokaler Reizung wird der Schirmer-Test [vgl. z.B. D. Vaughan und T. Asbury, Ophthalmologie, Springer, Berlin et al.

1983] durchgeführt:

Schirmer-Test
(Messzeit: 2 Minuten, Messungen in Millimeter)

Gruppe 1

| Tiernummer/ Geschlecht | vor dem Test | | nach 2 Wochen | | nach 4 Wochen | |
|---|---|---|---|---|---|---|
| | links | rechts | links | rechts | links | rechts |
| 389/m | 7 | 6 | 9 | 6 | 6 | 5 |
| 390/m | 3 | 5 | 8 | 5 | 5 | 6 |
| 391/m | 4 | 9 | 4 | 5 | 4 | 7 |
| 395/w | 5 | 4 | 4 | 7 | 5 | 6 |
| 396/w | 7 | 11 | 8 | 6 | 6 | 8 |
| 397/w | 5 | 8 | 4 | 7 | 4 | 5 |
| Mittel | 5,2 | 7,2 | 6,2 | 6,0 | 5,0 | 6,2 |

Gruppe 2

| Tiernummer/ Geschlecht | vor dem Test | | nach 2 Wochen | | nach 4 Wochen | |
|---|---|---|---|---|---|---|
| | links | rechts | links | rechts | links | rechts |
| 392/m | 8 | 7 | 3 | 6 | 2 | 8 |
| 393/m | 8 | 10 | 6 | 5 | 10 | 9 |
| 394/m | 5 | 4 | 5 | 7 | 4 | 9 |
| 398/w | 7 | 10 | 5 | 12 | 5 | 6 |
| 399/w | 4 | 8 | 7 | 6 | 5 | 2 |
| 400/w | 5 | 5 | 3 | 6 | 4 | 7 |
| Mittel | 6,2 | 7,3 | 4,8 | 7,0 | 5,0 | 6,8 |

m = männlich

w = weiblich

Beobachtungen und Messergebnisse, die mit dem Schirmer-Test gewonnen werden, zeigen, dass keine Unterschiede zwischen behandelten Augen und Kontrollaugen auftreten, die in Verbindung mit den zu testenden Augentropfen gebracht werden können.

Die aufgetretenen Unterschiede können als individuell angesehen werden, ohne eine biologische oder

toxikologische Relevanz zu haben.

Zusammenfassend kann somit gesagt werden, dass die Anwendung über vier Wochen der in dieser Patentschrift als Formulierungsbeispiel 1 genannten' Diclofenac-Na enthaltenden Augentropfenlösung am Kaninchenauge sicher ist. Mit dieser Untersuchung wird zugleich auch die lokale Verträglichkeit der Hilfsstoffe nachgewiesen.

Für den Zusatz Trometamol wurde ergänzend eine 7-tägige Augenreizwirkungs-Studie am Kaninchen durchgeführt. Es wird wieder mit zwei Gruppen zu jeweils sechs Tieren gearbeitet:

| Versuchsgruppe | 1 | | 2 | |
|---|---|---|---|---|
| Dosierung | 1) | | 2) | |
| Geschlecht | m | w | m | w |
| Zahl der Versuchstiere | 3 | 3 | 3 | 3 |
| Von der Norm abweichende Beobachtungen und Befunde | keine | keine | keine | keine |

1) Gruppe 1 linkes Auge: NaCl-Lösung 0,9 %
rechtes Auge: 0,5 % Trometamol

2) Gruppe 2 linkes Auge: Puffer
rechtes Auge: 2 % Trometamol

Die Augen werden zweimal täglich, vor der ersten und nach der letzten Applikation untersucht. Als Ergebnis kann zusammengefasst werden, dass Trometamol am Kaninchenauge eine gute Verträglichkeit aufweist.

Die im Tierversuch bewiesene gute Verträglichkeit konnte auch im Rahmen klinischer Verträglichkeitsprüfungen am Menschen gezeigt werden. 221 Personen werden mit Augentropfen, die dem Formulierungsbeispiel 1 entsprachen, behandelt. Ausser einem gelegentlichen, kurzdauernden, leichten Brennen direkt nach der Applikation werden keine spezifischen Nebenwirkungen beobachtet.

Insgesamt lassen die Ergebnisse somit den Schluss zu, dass es sich bei der vorliegenden Formulierung um eine lokal gut verträgliche Lösung zur ophthalmologischen Anwendung handelt.

## Patentansprüche

## Patentansprüche für folgende Vertragsstaaten : BE, CH, FR, GB, IT, LI, LU, NL, SE

1. Arzneimittel zur Behandlung von Entzündungen im Auge, enthaltend eine wässrige Lösung von Diclofenac-Natrium, einen Puffer, ein Isotonisierungsmittel, einen Lösungsvermittler und ein Konservierungsmittel, dadurch gekennzeichnet, dass es als Stabilisator für den Wirkstoff und das Konservierungsmittel 2-Amino-2-hydroxymethyl-1,3-propandiol oder ein Homologes davon mit bis zu 10 C-Atomen enthält.

2. Arzneimittel nach Anspruch 1, dadurch gekennzeichnet, dass es 2-Amino-2-hydroxymethyl-1,3-propandiol (Trometamol) als Stabilisator enthält.

3. Arzneimittel nach Anspruch 1 bis 2, dadurch gekennzeichnet, dass es den Stabilisator in einer Menge von 0,05 bis 5 % enthält.

4. Arzneimittel nach Anspruch 1 bis 3, dadurch gekennzeichnet, dass es eine 0,01 bis 0,15%ige wässrige Lösung von Diclofenac-Natrium enthält.

5. Arzneimittel nach Anspruch 1 bis 4, dadurch gekennzeichnet, dass es als Konservierungsmittel das Natriumsalz von 2-(Ethylmercurithio)-benzoesäure (Thiomersal) enthält.

6. Arzneimittel nach Anspruch 1 bis 4, dadurch gekennzeichnet, dass es als Konservierungsmittel Benzalkoniumchlorid enthält.

7. Arzneimittel nach Anspruch 1 bis 4, dadurch gekennzeichnet, dass es als Konservierungsmittel Cetylpyridiniumchlorid enthält.

8. Arzneimittel nach Anspruch 1, dadurch gekennzeichnet, dass es eine 0,05 bis 0,11%ige wässrige Lösung von Diclofenac-Natrium, als Puffer und Isotonisierungsmittel Borsäure in einer Menge von 1,9 %, als Lösungsvermittler ein Reaktionsprodukt aus Rizinusöl und Ethylenoxid in einer Menge von 5,0 %, als Konservierungsmittel das Natriumsalz von 2-(Ethylmercurithio)-benzoesäure (Thiomersal) in einer Menge von 0,002 bis 0,005 % und als Stabilisator 2-Amino-2-hydroxymethyl-1,3-propandiol (Trometamol) in einer Menge von 0,1 bis 1,0 % enthält.

9. Verfahren zur Herstellung eines Arzneimittels gemäss den Ansprüchen 1-8, dadurch gekennzeichnet, dass alle Komponenten des Arzneimittels unter sterilen Bedingungen homogen vermischt und abgefüllt werden.

**Patentansprüche für folgende Vertragsstaaten: ES, GR**

1. Verfahren zur Herstellung eines Arzneimittels für die Behandlung von Entzündungen im Auge, das eine wässrige Lösung von Diclofenac-Natrium, einen Puffer, ein Isotonisierungsmittel, einen Lösungsvermittler, ein Konservierungsmittel und als Stabilisator für den Wirkstoff und das Konservierungsmittel 2-Amino-2-hydroxymethyl-1,3-propandiol oder ein Homologes davon mit bis zu 10 C-Atomen enthält, dadurch gekennzeichnet, dass alle Komponenten des Arzneimittels unter sterilen Bedingungen homogen vermischt und abgefüllt werden.

2. Verfahren nach Anspruch 1, dadurch gekennzeichnet, dass das Arzneimittel 2-Amino-2-hydroxymethyl-1,3-propandiol (Trometamol) als Stabilisator enthält.

3. Verfahren nach Anspruch 1 bis 2, dadurch gekennzeichnet, dass das Arzneimittel den Stabilisator in einer Menge von 0,05 bis 5 % enthält.

4. Verfahren nach Anspruch 1 bis 3, dadurch gekennzeichnet, dass das Arzneimittel eine 0,01 bis 0,15%ige wässrige Lösung von Diclofenac-Natrium enthält.

5. Verfahren nach Anspruch 1 bis 4, dadurch gekennzeichnet, dass das Arzneimittel als Konservierungsmittel das Natirumsalz von 2-(Ethylmercurithio)-benzoesäure (Thiomersal) enthält.

6. Verfahren nach Anspruch 1 bis 4, dadurch gekennzeichnet, dass das Arzneimittel als Konservierungsmittel Benzalkoniumchlorid enthält.

7. Verfahren nach Anspruch 1 bis 4, dadurch gekennzeichnet, dass das Arzneimittel als Konservierungsmittel Cetylpyridiniumchlorid enthält.

8. Verfahren nach Anspruch 1, dadurch gekennzeichnet, dass das Arzneimittel eine 0,05 bis 0,11%ige wässrige Lösung von Diclofenac-Natrium, als Puffer und Isotonisierungsmittel Borsäure in einer Menge von 1,9 %, als Lösungsvermittler ein Reaktionsprodukt aus Rizinusöl und Ethylenoxid in einer Menge von 5,0 %, als Konservierungsmittel das Natriumsalz von 2-(Ethylmercurithio)-benzoesäure (Thiomersal) in einer Menge von 0,002 bis 0,005 % und als Stabilisator 2-Amino-2-hydroxymethyl-1,3-propandiol (Trometamol) in einer Menge von 0,1 bis 1,0 % enthält.

**Claims**

**Claims for the following Contracting States : BE, CH, FR, GB, IT, LI, LU, NL, SE**

1. A medicament for the treatment of inflammations of the eye, comprising an aqueous solution of

diclofenac sodium, a buffer, an isotonising agent, a solution aid and a preservative, characterised in that it comprises 2-amino-2-hydroxymethyl-1,3-propanediol or a homologue thereof having up to 10 carbon atoms as stabiliser for the active ingredient and the preservative.

**2.** A medicament according to claim 1, characterised in that it comprises 2-amino-2-hydroxymethyl-1,3-propanediol (trometamol) as stabiliser.

**3.** A medicament according to claims 1 and 2, characterised in that it comprises the stabiliser in an amount of from 0.05 to 5 %.

**4.** A medicament according to claims 1 to 3, characterised in that it comprises a 0.01 to 0.15 % aqueous solution of diclofenac sodium.

**5.** A medicament according to claims 1 to 4, characterised in that it comprises the sodium salt of 2-(ethylmercurithio)-benzoic acid (thiomersal) as preservative.

**6.** A medicament according to claims 1 to 4, characterised in that it comprises benzalkonium chloride as preservative.

**7.** A medicament according to claims 1 to 4, characterised in that it comprises cetylpyridinium chloride as preservative.

**8.** A medicament according to claim 1, characterised in that it comprises a 0.05 to 0.11 % aqueous solution of diclofenac sodium, boric acid in an amount of 1.9 % as buffer and isotonising agent, a reaction product of castor oil and ethylene oxide in an amount of 5.0 % as solution aid, the sodium salt of 2-(ethylmercurithio)-benzoic acid (thiomersal) in an amount of from 0.002 to 0.005 % as preservative and 2-amino-2-hydroxymethyl-1,3-propanediol (trometamol) in an amount of from 0.1 to 1.0 % as stabiliser.

**9.** A process for the manufacture of a medicament according to claims 1 to 8, characterised in that all of the components of the medicament are mixed homogeneously, and filled, under sterile conditions.

**Claims for the following Contracting States : ES, GR**

**1.** A process for the manufacture of a medicament for the treatment of inflammations of the eye, which medicament comprises an aqueous solution of diclofenac sodium, a buffer, an isotonising agent, a solution aid, a preservative, and 2-amino-2-hydroxymethyl-1,3-propanediol or a homologue thereof having up to 10 carbon atoms as stabiliser for the active ingredient and the preservative, characterised in that all of the components of the medicament are mixed homogeneously, and filled, under sterile conditions.

**2.** A process according to claim 1, characterised in that the medicament comprises 2-amino-2-hydroxymethyl-1,3-propanediol (trometamol) as stabiliser.

**3.** A process according to claims 1 and 2, characterised in that the medicament comprises the stabiliser in an amount of from 0.05 to 5 %.

**4.** A process according to claims 1 to 3, characterised in that the medicament comprises a 0.01 to 0.15 % aqueous solution of diclofenac sodium.

**5.** A process according to claims 1 to 4, characterised in that the medicament comprises the sodium salt of 2-(ethylmercurithio)-benzoic acid (thiomersal) as preservative.

**6.** A process according to claims 1 to 4, characterised in that the medicament comprises benzalkonium chloride as preservative.

**7.** A process according to claims 1 to 4, characterised in that the medicament comprises cetylpyridinium chloride as preservative.

8. A process according to claim 1, characterised in that the medicament comprises a 0.05 to 0.11 % aqueous solution of diclofenac sodium, boric acid in an amount of 1.9 % as buffer and isotonising agent, a reaction product of castor oil and ethylene oxide in an amount of 5.0 % as solution aid, the sodium salt of 2-(ethylmercurithio)-benzoic acid (thiomersal) in an amount of from 0.002 to 0.005 % as preservative and 2-amino-2-hydroxymethyl-1,3-propanediol (trometamol) in an amount of from 0.1 to 1.0 % as stabiliser.

**Revendications**

**Revendications pour les Etats contractants suivants : BE, CE, FR, GB, IT, LI, LU, NL, SE**

1. Médicament pour le traitement des inflammations oculaires contenant une solution aqueuse de Diclofénac sodique, un tampon, un agent d'isotonisation, un tiers-solvant et un agent de conservation, caractérisé en ce qu'elle contient comme stabilisateur pour la substance active et comme agent de conservation le 2-amino-2-hydroxyméthyl-1,3-propanediol ou un de ses homologues ayant jusqu'à 10 atomes de carbone.

2. Médicament selon la revendication 1, caractérisé en ce qu'il contient le 2-amino-2-hydroxyméthyl-1,3-propanediol (Trométamol) comme stabilisateur.

3. Médicament selon les revendications 1 et 2, caractérisé en ce qu'il contient le stabilisateur en une quantité de 0,05 à 5%.

4. Médicament selon l'une des revendications 1 à 3, caractérisé en ce qu'il contient une solution aqueuse à 0,01 à 0,15% de Diclofénac sodique.

5. Médicament selon les revendications 1 à 4, caractérisé en ce qu'il contient comme agent de conservation le sel de sodium de l'acide 2-(éthylmercurithio)-benzoïque (Thiomersal).

6. Médicament selon les revendications 1 à 4, caractérisé en ce qu'il contient comme agent de conservation le chlorure de benzalconium.

7. Médicament selon les revendications 1 à 4, caractérisé en ce qu'il contient comme agent de conservation le chlorure de cétylpyridinium.

8. Médicament selon la revendication 1, caractérisé en ce qu'il contient une solution aqueuse à 0,05 à 0,11% de Diclofénac sodique, comme tampon et agent d'isotonisation de l'acide borique, en une quantité de 1,9%, comme tiers solvant un produit de réaction d'huile de ricin et d'oxyde d'éthylène en une quantité de 5,0%, comme agent de conservation, le sel de sodium de l'acide 2-(éthylmercurithio)-benzoïque (Thiomersal) en une quantité de 0,002 à 0,005% et comme stabilisateur le 2-amino-2-hydroxyméthyl-1,3-propanediol (Trométamol) en une quantité de 0,1 à 1,0%.

9. Procédé de préparation d'un médicament selon les revendications 1-8, caractérisé en ce que tous les composants du médicament sont mélangés et versés de façon homogène dans des conditions stériles.

**Revendications pour les Etats contractants suivants : ES, GR**

1. Procédé de préparation d'un médicament pour le traitement des inflammations oculaires, qui contient une solution aqueuse de Diclofénac sodique, un tampon, un agent d'isotonisation, un tiers-solvant, un agent de conservation, et comme stabilisateur pour la substance active et l'agent de conservation le 2-amino-2-hydroxyméthyl-1,3-propanediol ou l'un de ses homologues ayant jusqu'à 10 atomes de carbone, caractérisé en ce que tous les composants du médicament sont mélangés et versés de façon homogène dans des conditions stériles.

2. Procédé la revendication 1, caractérisé en ce que le médicament contient le 2-amino-2-hydroxyméthyl-1,3-propanediol (Trométamol) comme stabilisateur.

3. Procédé selon les revendications 1 et 2, caractérisé en ce que le médicament contient le stabilisateur en une quantité de 0,05 à 5%.

4. Procédé selon les revendications 1 à 3, caractérisé en ce que le médicament contient une solution aqueuse à 0,01 à 0,15% de Diclofénac sodique.

5. Procédé selon les revendications 1 à 4, caractérisé en ce que le médicament contient comme agent de conservation un sel de sodium de l'acide 2-(éthylmercurithio)-benzoïque (Thiomersal).

6. Procédé selon les revendications 1 à 4, caractérisé en ce que le médicament contient comme agent de conservation le chlorure de benzalconium.

7. Procédé selon les revendications 1 à 4, caractérisé en ce que le médicament contient comme agent de conservation le chlorure de cétylpyridinium.

8. Procédé selon la revendication 1, caractérisé en ce que le médicament contient une solution aqueuse à 0,05 à 0,11% de Diclofénac sodique, comme tampon et agent d'isotonisation de l'acide borique en une quantité de 1,9%, comme tiers-solvant un produit de réaction d'huile de ricin et d'oxyde d'éthylène en une quantité de 5,0%, comme agent de conservation le sel de sodium de l'acide 2-(éthylmercurithio)-benzoïque (Thiomersal) en une quantité de 0,002 à 0,005%, et comme stabilisateur le 2-amino-2-hydroxyméthyl-1,3-propanediol (Trométamol) en une quantité de 0,1 à 1,0%.